# EUROPEAN PATENT SPECIFICATION

(11) **EP 2 773 268 B1**
(45) Date of publication and mention of the grant of the patent: **17.02.2021**
(21) Application number: 12844797.6
(22) Date of filing: 02.11.2012
(51) Int. Cl.: A61B 8/08, G01N 29/24, A61B 5/00, G01N 21/17, A61B 5/1455, C03B 37/15, G02B 6/36

(54) **HANDHELD OPTOACOUSTIC PROBE**
TRAGBARE OPTOAKUSTISCHE SONDE
SONDE OPTOACOUSTIQUE PORTATIVE

(30) Priority: 02.11.2011 US 201113287759
(43) Date of publication of application: 10.09.2014
(73) Proprietor: Seno Medical Instruments, Inc., San Antonio, TX 78230 (US)
(72) Inventor: HERZOG, Donald, G., Collingswood, NJ 08108 (US); MILLER, Thomas, G., Houston, TX 77079 (US)
(74) Representative: Leach, Sean Adam
(86) International application number: PCT/US2012/063245
(87) International publication number: WO 2013/067304

(56) References cited:
- EP-A1- 0 282 234
- WO-A1-01/10295
- WO-A2-2010/045421
- US-A- 4 651 850
- US-A- 5 348 002
- US-A- 5 840 023
- US-A1- 2010 016 717
- US-A1- 2011 201 914
- YAMASHITA Y ET AL: "Low Sound Velocity and Acoustic Attenuation Silicone Rubber Lens Based on Nano-Powder-Composite for Medical Echo Ultrasound Array Probes", APPLICATIONS OF FERROELECTRICS, 2007. ISAF 2007. SIXTEENTH IEEE INTERN ATIONAL SYMPOSIUM ON, IEEE, PI, 31 May 2007 (2007-05-31), pages 752-753, XP031167482, ISBN: 978-1-4244-1333-1
- INADA K: "BASIC COMPONENETS AND FIBER OPTIC PASSIVE COMPONENTS: STATUS AND TRENDS IN JAPAN", IEEE JOURNAL ON SELECTED AREAS IN COMMUNICATIONS, IEEE SERVICE CENTER, PISCATAWAY, US, vol. SAC-04, no. 4, 1 July 1986 (1986-07-01), pages 472-479, XP000877018, ISSN: 0733-8716

## Description

### FIELD

The present invention relates in general to the field of medical imaging, and in particular to an optoacoustic probe for use in medical imaging.

An optoacoustic probe according to the state of the art is for instance described in US2010/0016717A1.

### BRIEF DESCRIPTION OF THE DRAWINGS

Objects, features, and advantages of the present disclosure will be apparent from the following description of preferred embodiments as illustrated in the accompanying drawings, in which reference characters refer to the same parts throughout the various views. The drawings are not necessarily to scale, emphasis instead being placed upon illustrating principles of the invention.
FIG. 1 shows a schematic block diagram illustrating an embodiment of a combined optoacoustic and ultrasound system that may be used as a platform for the methods and devices disclosed herein.
FIG. 2 shows a schematic orthogonal view of an embodiment of a probe that may be used in connection with the methods and other devices disclosed herein.
FIG. 3 shows an exploded view of an embodiment of the probe shown in Figure 2.
FIG. 4 shows a cutaway view taken along the centerline of the wider side of the probe shown in Figure 2.
FIG. 5a is a side-view not-to-scale diagrammatic two dimensional representation of light exiting an optical fiber.
FIG. 5b shows an end view of a light pattern that may result on a surface from placement of optical fibers directly on to that surface.
FIG. 6a shows an end view of a desirable light pattern for use in connection with the optoacoustic techniques discussed herein.
FIG. 6b shows a side view diagrammatic representation of an effect of a ground glass beam expander on the light emitting from a fiber shown in Figure 5a.
FIG. 6c shows a side view diagrammatic representation of an effect of a concave lens beam expander on the light emitting from a fiber shown in Figure 5a.

### DETAILED DESCRIPTION

Reference will now be made in detail to various embodiments, examples of which are illustrated in the accompanying drawings.

Generally, device 100 provides an optoacoustic system that may also be employed as a multi-modality, combined optoacoustic and ultrasound system. In an embodiment, the device 100 includes a probe 102 connected via a light path 132 and an electrical path 108 to a system chassis 101. Within the system chassis 101 is housed a light subsystem 129 and a computing subsystem 128. The computing subsystem 128 includes one or more computing components for ultrasound control and analysis and optoacoustic control and analysis; these components may be separate, or integrated. In an embodiment, the computing subsystem comprises a relay system 110, an optoacoustic processing and overlay system 140 and an ultrasound instrument 150.

The light system 129 is capable of producing pulses of light of at least two different wavelengths. In an embodiment, the light system 129 output should be capable of producing short pulses of light in each of those wavelengths, e.g., a pulse lasting less than about 100 ns, and more preferably around 5 ns. As will be apparent to one of ordinary skill in the art from this disclosure, the inventions disclosed herein may also be practiced using pulsed light comprising pulses lasting greater than 100 ns. In an embodiment, the light source 129 includes two separate lights 130, 131. The output of the light system 129 is delivered to the probe 102 via the optical path 132. In an embodiment, the lights 130, 131 are lasers producing light in the infrared, near-infrared, and/or visible spectrum. In an embodiment, light 130 and light 131 each produce light at a different wavelength in the infrared or near-infrared spectrum. In an embodiment, the optical path 132 used to deliver light from the light source 129 to the probe 102 is a fiber optic bundle comprising multiple strands of optical fiber. In an embodiment, the optical path 132 comprises sufficient optical fibers of sufficient size (diameter) to carry a short, high powered pulse of light to the distal end of the optical path 132. In an embodiment, the total pulse energy carried over the optical path 132 may be on the order of one or more millijoules. In an embodiment, the total energy per light pulse carried over the optical path 132 is less than about 100 millijoules. In an embodiment, the total energy per light pulse carried over the optical path 132 is in the range of about 10-30 millijoules, and the optical path 132 comprises around 1,000 optical fibers of about 150 microns each. In an embodiment, a single fiber can be used as the optical path. In such embodiment, the fiber may be 1000-1500 microns in diameter. Of course, the diameter of such single fiber may be smaller, e.g., 400 microns. Given the required total pulse energy carried over the fiber, one skilled in the art can calculate the diameter required of the fiber accordingly.

In an illustrative embodiment, the light system 129 may use Nd-YAG and Alexandrite lasers as its two lights 130, 131, although other types, and additional lights, may also be used. Lights 130, 131 should be capable of producing a short pulse of light, e.g., a pulse lasting less than about 100 ns, and more preferably around 5 ns. In an embodiment, the two lights 130, 131 can be separately triggered. In an embodiment, the light output by the lights 130, 131 may be projected onto the same light path 132 through the use of an optical element 133 that generally permits one light 130 to pass through from a first side to a second side, while reflecting one light 131 that strikes the second side. The use of optical element 133 or a similar element permits the alignment of the output of two lights 130, 131 such as lasers onto proximal end of the light path 132. In an embodiment, optical elements 133 can align the light output from more than two lasers, for example, through the use of multiple optical elements 133. In an embodiment, multiple light systems and light paths may be employed, with the light of each light system being carried on separate fibers that are intermingled at their distal ends.

Although the total energy per light pulse carried over the optical path is in the order of tens of millijoules, because the pulse of lights 130, 131 is so short, the peak power output over the optical path 132 is frequently approaching or in the megawatt range. Accordingly, the output of lights 130, 131 has the capacity to cause the optical fibers and/or the cladding on the optical fibers to burn. Burnt optical fibers and burnt cladding can exacerbate the problem as they begin to transmit less light power and cause more heating. Accordingly, in an embodiment, sufficient number and size optical fibers are present in the optical path 132 to permit handling of the peak power loads and avoid fiber burnout. To accommodate higher peak power, a larger fiber bundle can be used. It will be apparent to a person of skill in the art that the peak power capacity of a fiber bundle can be increased by increasing the number of optical fibers, or the diameter of optical fibers, or both. Notably, however, as the dimension of the fiber bundle increases, the weight and flexibility of the optical path 132 decreases. Moreover, when using more optical fibers, or optical fibers of a larger diameter, the output of light source 129 must be delivered to the optical path 132 across the wider diameter of the larger bundle. In an embodiment, regardless of the ultimate size of the proximal end of light path 132, the output of light source 129 should be distributed sufficiently across its cross section to prevent burn-out failures when operating in expected peak power ranges.

In an embodiment, the fibers of the proximal end of the light path 132 may be fused to form a fused entry point to the optical path 132 for the output of light source 129. In an embodiment, the fiber ends can be fused by applying heat. Once the proximal end of optical path 132 has been fused, it will resist burnout at substantially higher peak power. For example, using a fused end light path 132 may permit carriage of three, four or even five times as much peak power. The ability to carry substantially higher peak power in a given optical path 132 permits use of a more flexible and lighter fiber optic bundle to carry the same peak power as an un-fused optical path 132. Thus, in an embodiment, where a ½" fiber optic bundle may have been required in an un-fused bundle of optical fibers forming an optical path, a ¼" fiber optic bundle with a fused proximal end may be used to carry the same peak power. A ¼" fiber optic bundle with a fused proximal end is approximately ¼ of the weight and much more flexible than a ½" fiber optic bundle. Moreover, fusing of the proximal end of light path 132 may produce an even smaller fused area to illuminate using light source 132 as the fusing removes the inter-fiber spaces that would have existed in the bundled end of the round-cross-section optical fibers. Accordingly, one or more of the following advantages may be attained by fusing the proximal end of the optical fibers comprising the light path 132: reduced weight of the light path; increased flexibility of the light path; reduced failure; increased reliability; higher peak power capacity.

In an embodiment, the light output by the lights 130, 131 is sent towards a fused optical fiber bundle at the proximal end of light path 132 via an optical path, which may include optical element 133, internal to the light source 129. In an embodiment, light source 129 is a laser system capable of outputting laser light pulses, at one or a more wavelengths, onto light path 132. In an embodiment, light path 132 is a fiber optic bundle having a fused end proximal to the light source 129.

In an embodiment, the device 100 also comprises an electrical path 108 running to and/or from the probe 102 to a relay system 110 within the system chassis 101. The electrical path 108 may run near, alongside or coaxially with the optical path 132 from the probe 102 toward their respective connections on the system chassis 101. In an embodiment, the electrical path 108 comprises a plurality of separate coaxial wires. In an embodiment, the electrical path 108 is run in a common jacket with at least a portion of the optical path 132. Running electrical path 108 in a common jacket with at least a portion of the optical path 132 reduces the number of cables running from the system chassis 101 to the probe 102. Running electrical path 108 in a common jacket with at least a portion of the optical path 132 may minimize the diameter and weight of, and increase the durability of, the combined cables (i.e., optical path 132 and electrical path 108) running from the system chassis 101 to the probe 102.

In an embodiment, the plurality of coaxial wires are woven around at least a portion of the optical path 132. As discussed above, many considerations go into the number of separate optical fibers used in optical path 132. As discussed further below, numerous design considerations go into the number of separate electrical leads or traces forming the electrical path 108. In an embodiment, there are about 256 leads (corresponding to 256 transducers) forming the electrical path 108 and approximately 1,000 separate optical fibers forming the optical path 132, making the fiber:lead ratio about 4:1. As will be apparent, it is possible to comingle the optical fibers and leads or traces in the electrical path in a variety of ways, including, for example, bundling a group of individual fibers with a single electrical lead or trace, or bundling proportionally larger groupings of fibers and leads together. In an embodiment, the bundling of fibers and leads or traces would be done generally in the proportion of fibers:leads in the system.

One or more displays 112, 114, which may be touch screen displays, are provided for displaying images and all or portions of the device 100 user interface. One or more other user input devices (not shown) such as a keyboard, mouse and various other input devices (e.g., dials and switches) may be provided for receiving input from an operator. As an option, power and control signal lines 109 carry power to the probe 102 and control signals between the probe 102 and the computing subsystem 128.

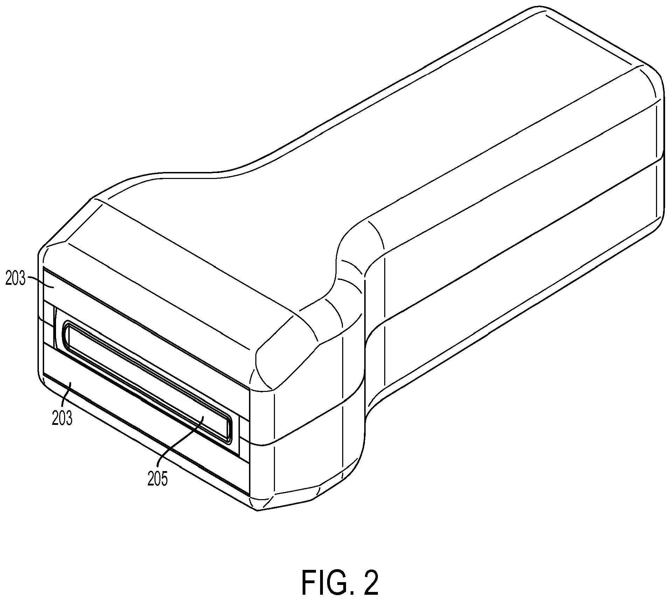

Turning now to Figure 2, the probe 102 includes an array of ultrasound transducer elements forming an ultrasound transducer (not shown) covered by an acoustic lens 205. In an embodiment the ultrasound transducer comprises an array of piezoelectric elements that can both transmit and receive acoustic energy. In an embodiment, at least some of the ultrasound transducer elements are capable of detecting ultrasound frequencies over a wide range. For example, ultrasound transducer elements may be capable of detecting ultrasound in the range from about 50 Khz to 20 Mhz. This range can be achieved by applying a high impedance load (e.g., in the range of 5,000 to 50,000 ohms) to achieve a lower frequency response. The ultrasound transducer elements are capable of generating electrical energy in response to receiving ultrasound acoustic energy. The electrical energy generated by the ultrasound transducer elements receiving ultrasound is transmitted to the computing subsystem 128 via electrical path 108.

The probe 102 also includes one or more optical windows 203 through which the light carried on optical path 132 can be transmitted to the surface of a three-dimensional volume 160. In an embodiment, it is desirable to locate one side of the optical window 203 as close as practical to the acoustic lens 205. The total area of an optical window 203 is important to maximize energy for a given fluence incident on the surface of the volume 160.

In an embodiment, the multiple strands of optical fiber making up the optical path 132 are terminated in two light bars (not shown). In an embodiment, the ultrasound transducer elements (not shown) are arranged in an array that runs along a geometric plane and are generally spaced equidistant from each other. In an embodiment, the light bars (not shown) are oriented longitudinally, on each side of the planar array of ultrasound transducer elements. Preferably the ultrasound transducer elements generate electrical energy in response to both ultrasound acoustic energy received in response to stimulation caused by the pulsed light sources 130, 131 and to ultrasound acoustic energy received in response to acoustic output of the ultrasound transducer elements.

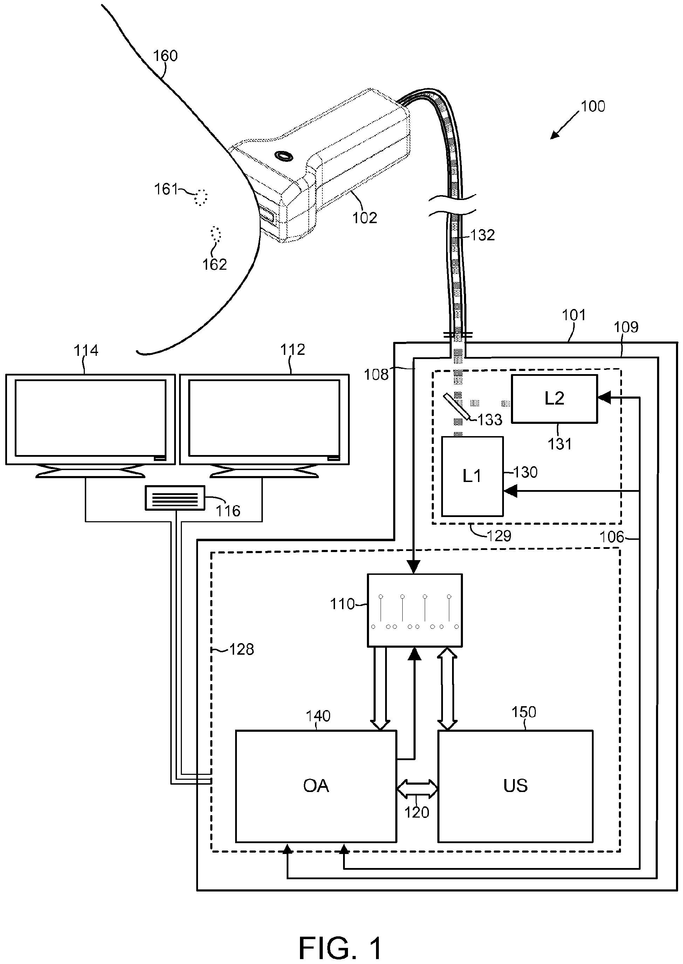

Referring back to Figure 1, in use, the probe 102 may be placed in close proximity with organic tissue, phantom or other three-dimensional volume 160 that may have one or more localized inhomogenities 161, 162, such as e.g., a tumor, within. An ultrasound gel (not shown) or other material may be used to improve acoustic coupling between the probe 102 and the surface of the volume 160. The probe 102, when in proximity with the surface of the volume 160, can emit a pulse of a light through the optical windows 203 or an ultrasound through acoustic lens 205, and then generate electrical energy corresponding to ultrasound detected in response to the emitted light or sound.

In an embodiment, the computing subsystem 128 can trigger activity from light system 129 over control signal line 106. In an alternative embodiment, the light system 129 can create the trigger signal and inform the computing subsystem 128 of its activity over control signal line 106. Such information can be used to by the computing subsystem 128 to begin the data acquisition process. In this respect, it is noted that communication over control signal line 106 can flow both ways between the computing subsystem 128 (and/or the optoacoustic processing and overlay system 140 therein) and the light system 129.

In an embodiment, computing subsystem 128 can utilize control signal line 106 to control the start time and duration of light pulses from each light source 130, 131. The computing subsystem 128 can also trigger the probe 102 to emit ultrasound acoustic energy via the ultrasound transducer elements behind the acoustic lens 205.

In an embodiment, the computing subsystem 128 receives electrical signals representative of the ultrasound detected by the ultrasound transducer elements, in response to an ultrasound transmitted signal or an optically generated ultrasound signal, behind the acoustic lens 205 via electrical path 108. In an embodiment, the electrical signal representative of the ultrasound detected by the ultrasound transducer elements behind the acoustic lens 205 is the analog electrical signal created by the elements themselves. In such embodiment, the electrical signals representative of the ultrasound detected by the ultrasound transducer elements behind the acoustic lens 205 is transmitted to the computing subsystem via electrical path 108, and electrical path 108 is selectively directed by relay system 110 to the optoacoustic processing and overlay system 140 or the ultrasound instrument 150 for processing of the detected ultrasound. In such embodiment, the ultrasound instrument 150 can receive the same input (over the same connector) as it would receive from an ultrasound probe.

In another embodiment, the electrical signal representative of the ultrasound detected by the ultrasound transducer elements behind the acoustic lens 205 is digitized by an analog-to-digital converter which can be housed in the probe 102. In such embodiment, time-resolved electrical signal representative of the ultrasound detected by the ultrasound transducer elements behind the acoustic lens 205 is transmitted across the electrical path 108. Where the electrical signal is digitized at the probe 102, as will be apparent to one of skill in the art, the relay system 110 may be implemented to deliver digital data to the optoacoustic processing and overlay system 140 or the ultrasound instrument 150, or may not be needed at all.

The signal representative of the ultrasound detected by each of the plurality of ultrasound transducer elements behind the acoustic lens 205 may be carried on a separate wire over the electrical path 108. Alternatively, the signal representative of the ultrasound detected by a plurality of ultrasound transducer elements behind the acoustic lens 205, or even all of the ultrasound transducer elements behind the acoustic lens 205, may be multiplexed (e.g., time division or frequency division) utilizing a multiplexer in the probe and a demultiplexer in the computing subsystem 128.

In an embodiment, the ultrasound instrument 150 processes ultrasound-induced acoustic signals to produce ultrasound images and the optoacoustic processing and overlay system 140 processes light-induced acoustic signals to produce optoacoustic images. In an embodiment, the ultrasound instrument 150 and optoacoustic processing and overlay system 140 can be combined into an integrated system performing the combined functions of both. As discussed above, in an embodiment, electrical signals representative of ultrasound detected by the probe 102 and delivered to the computing subsystem 128 via electrical path 108 is switched between the ultrasound instrument 150 and the optoacoustic instrument 140 via relay system 110 in accordance with whether the signal results from ultrasound stimulation or light stimulation.

In an embodiment, tomographic images reflecting the ultrasound-stimulated data may be generated by the ultrasound instrument 150 and tomographic images reflecting the light-stimulated data may be generated by the optoacoustic processing and overlay system 140.

Images, including tomographic images, produced by the optoacoustic processing and overlay system 140 can be stored in a computer memory in that system, along with data associated with sequence or time and date of the image data that was captured. Images, including tomographic images, produced by the ultrasound instrument 150 may be transmitted to the optoacoustic processing and overlay system 140 via a suitable interface 170, where they can be stored, along with images generated from the light-stimulated data, in a time-synchronized manner. In an embodiment, images stored in the memory of the optoacoustic processing and overlay system 140 can be recorded to another memory, e.g., a non-volatile memory internal to, or external to, the device.

In an embodiment, the optoacoustic processing and overlay system 140 can overlay images produced by the ultrasound instrument with images produced by optoacoustic instrument 140 for storage in the memory and/or display on one or more monitors 112, 114. In an embodiment, the overlayed optoacoustic image may be shown in a distinct color to distinguish it from the ultrasound image. In an embodiment, the overlaid optoacoustic image may contain colors that correspond to details discernable through optoacoustic imaging, such as, for example, blood oxygenation. In an embodiment, oxygenated blood is shown more in red than blue, while deoxygenated blood is shown in more blue than red. As used herein, the expression overlaid includes merging of the image by mixing as well as traditional overlaying of the image.

In an embodiment, the device 100 may be configured to operate in a cycle comprising a sequence of successively generating and acquiring data relating to one of the device's modalities, i.e., ultrasound or optoacoustic. The minimum time spacing between operation of the device's modalities depends on the device 100 components and their ability to fully execute and recycle for use. In an embodiment, a user can select between a variety of preprogrammed cycles such as: ultrasound only; wavelength one only; wavelength two only; wavelength one and two; and multiple iterations of wavelength one and two followed by ultrasound. Other combinations will be apparent to one of skill in the art. In an embodiment, additional cycles can be added by the machine operator. In an embodiment, the data collection of an entire cycle is generally intended to be directed to substantially the same portion of volume 160 and to be accomplished in rapid succession. In an embodiment, the device 100 cycles are normally in the range of 1 to 50 per second, and more typically in the range of 2 to 20 per second, as discussed above. The maximum cycle frequency is limited only by the capabilities of the cycle and modalities.

In an embodiment, the displays 112, 114 of device 100 can be configured to show various information depending upon the selected operating cycles. In an embodiment, any display 112, 144 or portion of the display can show at least one of the following: an ultrasound only image; a first wavelength response only image; a second wavelength response only image; a combined first and second wavelength response image; and/or an overlay ultrasound image and a wavelength response or combined wavelength response image. The combined first and second wavelength image may comprise a differential or other combinatorial means to provide the image. In an embodiment, an image can be displayed corresponding to each of the separate data collections in a cycle, or corresponding to the sum or difference between any or all of them.

In an embodiment, the device can be operated using a three-phase data collection operation, one phase generating and collecting data in response to ultrasound stimulus, one phase generating and collecting data in response to a first wavelength of light, and one phase generating and collecting data in response to a second wavelength of light.

Using proper wavelength(s), optoacoustics is effective in identifying blood within a volume 160, and using multiple wavelengths can be used to readily distinguish between oxygenated and deoxygenated blood. Similarly, using proper wavelengths, optoacoustics is effective for measuring localized hemoglobin content within a volume 160. Thus, for example, a malignant tumor, which is characterized by increased blood concentration and decreased oxygenation, will appear very differently in an optoacoustic image than a benign growth, which is not characterized by such an increased blood concentration and has more normal oxygenation. Moreover, specific wavelengths of light can be selected to better distinguish between various biological tissues and organs. While a large spectrum of infrared, near-infrared and visible wavelengths can produce optoacoustic response in biological entities, oxygenated blood is more optoacoustically responsive than deoxygenated blood to a light source having a wavelength of about 1064 nm, while deoxygenated blood is more optoacoustically responsive than oxygenated blood to a light source having a wavelength of 757 nm. The number and specific wavelength(s) of light used in the device 100 are selected in accordance with the makeup of the volume and the type of target that is of interest.

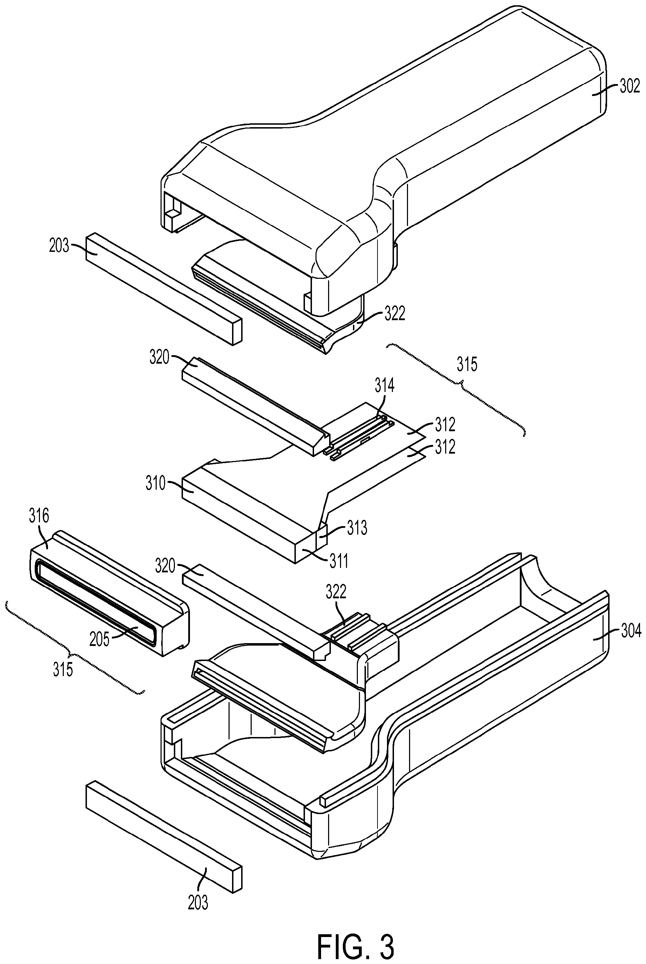

Figure 3 shows an exploded view of an embodiment of the probe 102 shown in Figure 2. Shells 302, 304 are separated to show the components within the probe 102. The shells 302, 304 may be made from plastic or any other suitable material. The surfaces of the shells 302, 304 that may be exposed to light, and especially light generated by the light subsystem 129, are preferably both reflective (i.e., light colored) material and light scattering (i.e., having a scattering coefficient between 1 and 10). In an embodiment, the surfaces of the shells 302, 304 are highly reflective, i.e., more than 75% reflective. In an embodiment, the surfaces of the shells 302, 304 are very highly reflective, i.e., more than about 90% reflective. In an embodiment, the surfaces of the shells 302, 304 have low optical absorption, i.e., less than 25% absorptive. In an embodiment, the surfaces of the shells 302, 304 have very low optical absorption, i.e., less than about 10% absorptive. In addition, the material forming the shells 302, 304 should be acoustically absorbent to absorb, rather than reflect or transmit acoustic energy. In an embodiment, white plastic shells 302, 304 are used.

In an embodiment, flex circuit 312 comprises a plurality of electrical traces (not shown) connecting cable connectors 314 to an array of piezoelectric ultrasound transducer elements (not shown) forming ultrasound transducer 310. In an embodiment, flex circuit 312 is folded and wrapped around a backing 311, and may be secured thereto using a bonding agent such as silicon. In an embodiment, a block 313 is affixed to the backing 311 opposite the array of piezoelectric ultrasound transducer elements. In an embodiment, the ultrasound transducer 310 comprises at least 128 transducer elements, although it may be desirable to have a greater numbers of transducer elements, as additional elements may reduce distortion, and/or increase resolution, accuracy and/or depth of imaging of the device 100. The cable connectors 314 operatively connect the electrical traces, and thus, the ultrasound transducer 310, to the electrical path 108. In an embodiment, the electrical path 108 includes a coaxial wire for each ultrasound transducer element in the ultrasound transducer array 310.

The ultrasound transducer 310 fits within housing 316 so that the transducer elements are in close proximity to, or in contact with an acoustic lens 205. The acoustic lens 205 may comprise a silicon rubber, such as a room temperature vulcanization (RTV) silicon rubber. In an embodiment, the housing 316 and the acoustic lens 205 are formed as a single unit, from the same RTV silicon rubber material. In an embodiment, the ultrasound transducer 310, portions of the flex circuit 312, backing 311 and block 313 are secured within the housing 316 including an acoustic lens 205 using a suitable adhesive such as silicon to form a transducer assembly 315. The block 313 can be used to affix or secure the transducer assembly 315 to other components.

To whiten, and reduce the optoacoustic effect of light generated by the light subsystem 129 on an RTV silicon rubber acoustic lens 205 and/or the transducer assembly 315, in an embodiment, the RTV silicon rubber forming the acoustic lens 205 and/or the transducer assembly 315 may be doped with TiO₂. In an embodiment, the RTV silicon rubber forming the acoustic lens 205 and/or the transducer assembly 315 may be doped with approximately 4% TiO₂. In an embodiment, the outer surface of the acoustic lens 205 and/or the outer surface of the transducer assembly 315 may additionally be, or alternatively be, coated with a thin layer of metal such as brass, aluminum, copper or gold. Gold, however, has been found to have a tendency to flake or crack off of RTV silicon rubber. It has been found that the RTV silicon may be first coated with perylene, then coated with nickel, then coated with gold, and finally, again, coated with perylene. The multiple layering provides a durable gold coating without any substantial adverse effect to the acoustic properties of the acoustic lens 205, and without any substantial adverse effect to the transducer assembly 315 to detect ultrasound. In practice, it has been found that the perylene coatings beneath the nickel and over the gold layers, may curl at the edges rather than adhering well to the metals or rubber upon which it is deposited. Thus, as discussed in more detail below, in an embodiment, the portions of the acoustic lens 203 and/or transducer assembly 315 having a perylene coating edge are adapted to be mechanically secured against other components to prevent curling or peeling. In an embodiment, substantially the entire outer surface of the transducer assembly 315, including the acoustic lens 205, are coated with continuous layers of perylene, then nickel, then gold and then perylene again.

In an embodiment, a reflective material surrounds the transducer assembly 315 from the rear edge of the housing 316 to the end of the flex circuit 312 to reflect any light from the light path 132 that may be incident upon its surfaces. In an embodiment, an electromagnetic shield for RF energy surrounds the transducer assembly 315 from the rear edge of the housing 316 to the end of the flex circuit 312. In an embodiment, the lights 130, 131, may draw substantial energy (e.g., more than 1,000 volts for a few nanoseconds) creating substantial electromagnetic RF energy in the area of the probe 102. In an embodiment, the transducer assembly 315 from the rear edge of the housing 316 to the end of the flex circuit 312 is surrounded by a foil, which may act as a reflective material and an RF energy shield. In an embodiment, the foil is selected from the group: copper, gold, silver. In an embodiment, the foil is tied into the device's 100 electrical ground.

Spacers 320 space and position the light bar guide 322 with respect to the transducer assembly 315. Spacers are preferably made from materials that reduce its optoacoustic response to light generated by the light subsystem 129. In an embodiment, the spacers 320 are made from a material similar to the light contacting portions of the shells 302, 304. In an embodiment, the light bar guide 322 encases optical fibers that are part of the light path 132. In an embodiment, the optical fibers making up the light path 132 may be randomly (or pseudo-randomly) distributed throughout the light bar guide 322, thus making specific locations on the light receiving end of the fiber optic bundle at least pseudo-random with respect to corresponding specific locations on the light emitting end of the optical fibers retained by the light bar guide 322. As used herein the term randomly (or pseudo-randomly) distributed optical fibers making up the light path 132 means that the mapping of fibers from the proximal end to the distal end is done such that a localized interference in the light path 132 (e.g., burnout of a group of adjacent optical fibers) or a localized phenomenon (e.g., non-uniform light at the entry point to the optical path 132) will have an effect on the overall power transmitted, but will not have an operationally significant effect on any specific part of the distal end of the light path 132. Thus, two optical fibers adjacent at the proximal end are unlikely to be adjacent at the distal end of the optical path 132. Where optical fiber bundles are fused at the proximal and distal ends, the randomization must be done before at least one end is fused. As used herein the term randomly (or pseudo-randomly) distributed optical fibers does not mean that two different optical paths 132 - i.e., for different devices 100 - must differ from each other. In other words, a single "random" mapping may be reproduced in the light path of different devices 100 while still meeting the criteria of being a randomized. Because light generally behaves in a Gaussian manner, the entry point to the light path 132 is typically less than perfectly uniform. Randomization, as discussed above, may accommodate for the non-uniform entry of light into the light path 132. Randomization may also provide homogenization of light fluence over area illuminated, as it may aid in more evenly distributing the light fluence.

In an embodiment, the optical fibers encased by a light bar guide 322 all end on substantially the same geometric surface, e.g., a curved or flat plane. In one embodiment, after the fibers have been attached to the light bar guide 322, the fiber ends may be lapped and polished to provide for a more uniform angle of light emission. In an embodiment, the light bar guide 322, as installed in the assembled probe 102, directs the light emitting there-from at an angle slightly less than normal to the distal face of the probe 102, and specifically, at small angle inwards, towards the plane normal to and intersecting the center of the acoustic transducer array 310. In an embodiment, the distal end(s) of the optical path 132 should match - or closely approximate the shape of the acoustic transducer array 132.

The term bar, as used in "light bar guide" herein is not intended to import a specific shape. For example, the light bar guide 322 may guide the distal ends of optical fibers into substantially any shape such as, without limitation, a whole or part of a circle, oval, triangle, square, rectangle or any irregular shape.

In an embodiment, one or more light bar guides 322 and optical windows 203 are external to the shells 302, 304 housing the acoustic transducer assembly 315, and are adapted to be attached to the outer sides of one or more of the shells 302, 304.

In an embodiment, the angle of light emitting from the optical window 203 may be adjustable. In an embodiment, the light emitting from the optical window 203 may be adjustable across a range. At one end of the range, light may emit from the optical window 203 in a direction normal to the distal face of the probe 102, and at the other end of the range light may emit from the optical window 203 at an inward angle of up to 45 degrees or more towards the plane normal to and intersecting the center of the acoustic transducer array 310. The range can be smaller or larger.

In an embodiment wherein a probe has two optical windows 203, the angle of light emitting from both optical windows 203 can be adjustable, individually, or together. Where adjusting the angle of light emitting from both optical windows 203 together, the light direction would, in each case increase or decrease the angle of inward projection, that is, projection towards the plane normal to and intersecting the center of the acoustic transducer array 310. In this manner, a larger light fluence can be directed deeper into the volume 160 (by angling toward normal), or shallower (by angling more inwardly).

Controlling the direction of the light angle can be done by moving the light guide 322, or it can be accomplished optically through the use of post-light path 132 optics. Optical solutions may include the use of one or more lenses and/or prisms to re-direct the light that has been transmitted through the light path 132. Re-directed light can be directed to illuminate a desired area, such as an area directly beneath the transducer elements 310. Controlling the direction of light transmitted by the probe 102 is useful to maintain safe and optimize the direction of the light with respect to the skin and the transducers.

Control line 109 may be used to send commands redirecting light and/or to report the actual direction of light at the time a light pulse is emitted from the light path 132. The angle of the light emitting from the optical window 203 may be important data to consider when interpreting acoustic information resulting from the light pulse.

In an embodiment, the device 100 can adjust the angle of incident laser light emitting from the probe 102. Adjustment of the angle of incident laser light emitting from the probe 102 may be carried out under the control of commands which may be sent via control line 109, or may be manually carried out. In an embodiment, a standoff may be used, e.g., to help direct incident laser light to the desired depth, or closer to the surface than can be achieved without a standoff. In an embodiment, the standoff is relatively transparent to both acoustic and light, and preferably to acoustics in the ultrasound range and light one or more of the wavelengths utilized by the light source 129. While the use of standoffs is known in ultrasound applications to aid in imaging of objects close to the surface of the volume 160 because ultrasound resolution lacks the capability to detect objects at a nominal distance from its transducers, the use of a standoff in the present application is for a different purpose, namely, to allow the light sources to be aimed directly under the transducer elements 310. In an embodiment, the standoff is separate from the probe 102, and placed between the volume 160, and the distal end of the probe 102 comprising the acoustic lens 205 and one or more optical windows 203. In an embodiment, the standoff may be integral to the probe, and may be move into place and withdrawn as desired.

Optical windows 203 may also be part of the probe 102 assembly. In an embodiment, the optical windows 203 is spaced from the end of the light bar guide 322, and thus, from the ends of the optical fibers making up the light path 132. The term optical window, as used here, is not limited to mechanically or optically flat optical matter, nor solely to transparent optical matter. Instead, the term is used to refer to an optical element that may or may not effect light passing there-through, but will permit at least a substantial portion of the light incident on the side of the window proximal to the light path 132 to exit the probe assembly 102 in a manner that is dependent on the properties of the optical element. In an embodiment, the optical window 203 may be transparent, which permits transmission of light, and specifically light emitted from the end of the light path 132, to volume 160 when the distal end of the probe 102 is in contact with or close proximity to that volume 160. In an embodiment, the optical window 203 may be translucent, permitting diffusion and transmission of light, and specifically light emitted from the end of the light path 132, to volume 160 when the distal end of the probe 102 is in contact with or close proximity to that volume 160. In an embodiment, the optical window 203 may be a lens, permitting the shaping and directing of light, and specifically light emitted from the end of the light path 132, to volume 160 when the distal end of the probe 102 is in contact with or close proximity to that volume 160.

In the assembled probe 102, one edge of the optical window 203 is in close proximity to, or in contact with, the transducer assembly 315. The proximity of the optical window 203 to the transducer assembly 315 allows light emitted from the optical window 203 to be emitted from a location close to the acoustic lens 205, and thus close to the plane of the transducer array 310.

In use, a coupling agent (e.g., gel) may be used to improve the acoustic contact between the distal end of probe 102 and the volume 160. If the coupling agent makes contact with the distal end of the optical fibers forming the light path 132, extraneous acoustic signal may be generated in response to light transmission over the light path 132. In an embodiment, the distal end of the probe 102, including optical window 203, mitigates the potential acoustic effect of a coupling agent in response to light emitting from the light path 132 by creating a gap between the coupling agent and the distal end of the optical fibers.

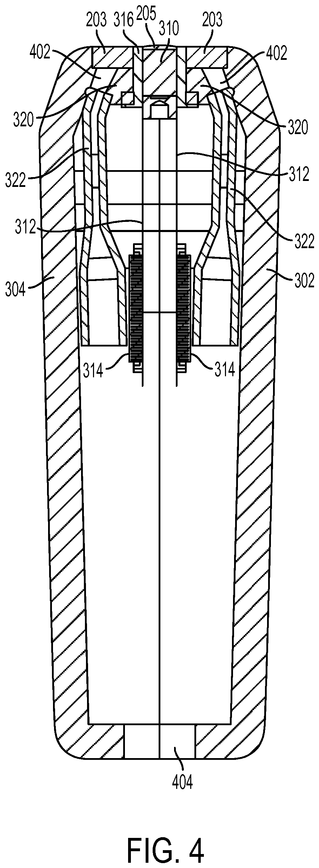

FIG. 4 shows a cutaway view taken along the centerline of the wider face of one embodiment of an assembled probe 102 such as the probe shown in Figure 2. Shells 302, 304 support optical windows 203 and transducer assembly 315 at the distal end of the probe 102. Spacers 320 supported by transducer assembly 315 and shells 302, 304 aid in the positioning of optical widows 203 and light bar guides 322, and in maintaining gap 402 between light bar guides 322 and the optical windows 203.

The distal ends of the optical fibers making up the light path 132 may be positioned such that they do not create a physical sound conduction path to the volume 160 or to the acoustic transducers 310. In an embodiment, the gap 402 serves the purpose of preventing high frequency sound conduction path between the distal ends of the optical fibers making up the light path 132 and the volume 160 or the acoustic transducers 310. Specially selected materials, as discussed below, can be used to ensure that the light bar guide 322 reduces and/or minimizes the physical sound conduction path between the distal end of the light path 132 and the volume 160 or the acoustic transducers 310.

Flex circuit 312, with piezoelectric transducer elements (not shown) thereon, wraps around backing 311, and electrically connects the piezoelectric transducer elements with the cable connectors 314 at each end of the flex circuit.

Opening 404 in the shells 302, 304 provides an opening for optical path 132 (Fig. 1), electrical path 108 (Fig. 1) and optional power and control lines 109 (Fig. 1) to enter the inside of the probe 102. In an embodiment, a rubber grommet (not shown) may be used to provide stability and strain relief to the paths or lines passing into the probe 102 through opening 404.

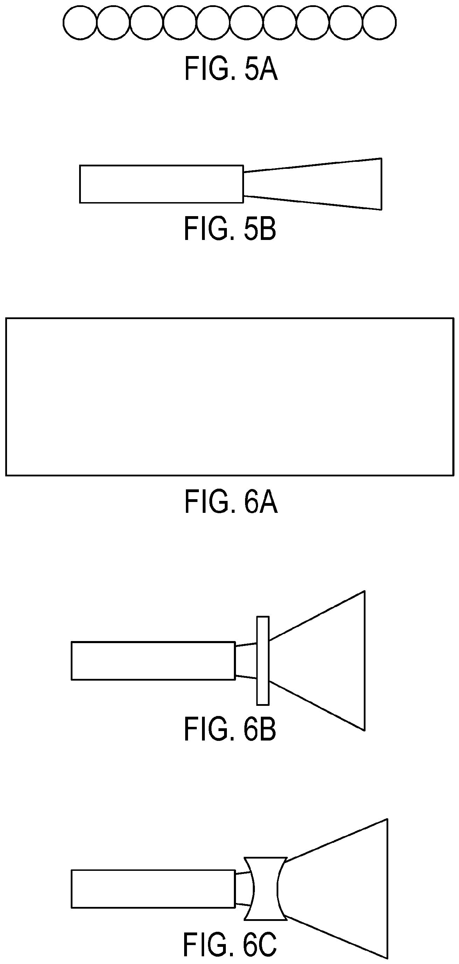

Turning to Figures 5a, a typical pattern of light striking a surface in close proximity to the ends of ten optical fibers is shown. Today, typical, reasonably flexible optical fibers have a diameter in the range of about 50 to 200 microns. Light exiting an optical fiber tends to expand slowly, see, for example, an illustrative example of light expanding after leaving the end of an optical fiber in Figure 5b. The rate of expansion of the light beam leaving an optical fiber is a function of the diameter of the optical fiber and the refraction index of the optical fiber material. When a group of optical fibers are placed in close proximity to a surface to be illuminated, a light pattern like that seen in Figure 5a results.

In an embodiment, optical fibers having smaller diameters are employed to broaden the illuminated area and minimize weight and increase flexibility of the light path 132. Light diverges as it exits a fiber optic, and its divergence as it exits is inversely related to the diameter of the fiber - in other words, light diverges faster out of smaller diameter fiber optics. Thus, for example, optical fibers in the range of under 50 microns, and potentially less than 30 microns may be desirable to broaden the illuminated area, thus reducing, or potentially eliminating the need for a beam expander. In an embodiment, the distal end of one or more groups of the optical fibers comprising the light path 132 may be fused to avoid the characteristic pattern of light shown in Figure 5a.

In an embodiment, an optoacoustic probe should produce a relatively uniform light distribution incident upon the surface of the illuminated volume. It may also be desirable for an optoacoustic probe to produce a relatively large area of light distribution. Providing a relatively large and uniform light distribution permits an optoacoustic probe to deliver a maximum amount of energy without exceeding a specific light fluence on any given area of the illuminated surface, which can maximize patient safety and/or improve the signal-to-noise ratio. For these reasons, it is not desirable to locate the optical fiber ends in too close proximity with the surface of the illuminated volume, and thus, obtain a small or uneven light distribution such as the one seen in Figure 5a.

In an embodiment, the optical fibers may be moved away from the surface of a volume to be illuminated. Moving the end of the optical fibers away from the surface of the volume to be illuminated will cause the beams emitted from each optical fiber to expand, and produce a more uniform area of light distribution. One potential issue associated with moving the optical fibers away from the surface of the volume to be illuminated, is the optoacoustic effects caused by stray portions of the expanding beam. Another potential issue is the effect of enlarging the distance (between the end of the optical fibers and the surface to be illuminated) on the shape or size of a probe. Further, increasing the number of optical fibers (and thus enlarging the area of the fiber bundle emitting light) will increase the cost, weight and flexibility of the optical path 132 (Figure 1), and may also affect the size of the probe.

Because the probe 102 is designed to be handheld, it is desirable to keep the probe head (the wider, distal portion of the probe 102) short so that the probe stem (the narrower, proximal portion of the probe 102) is relatively close to the surface of volume 160. Additionally, because the probe 102 is designed to be handheld, its total thickness is also a consideration for comfort, convenience and operational effectiveness. Accordingly, locating the distal ends of the fibers forming light path 132 at a sufficient distance from the optical window 203 to permit expansion to fill the optical windows 203 with uniform light fluence is not preferred. Similarly, using a very large number of fibers to enlarge the area of the fiber bundle held by the light bar guide 322 at the distal end of the light path 132 and thereby attempting to permit expansion to fill the optical windows 203 with uniform light fluence is also not preferred as it would, among other things cause undue weight, inflexibility, size and cost. Moreover, reducing the size of the optical window 203 would reduce the total potential safe energy output of the device, and thus, is not preferred.

Turning to Figures 6b and 6c, in an embodiment, a beam expander 601b, 601c may be used to expand the beam of light, causing it to become more uniform over a shorter distance. Figure 6b shows the use of a ground or frosted glass beam expander 601b, while Figure 6c shows the use of a lens beam expander 601c. In an embodiment, where the light bar guide 322 is generally rectangular, a lens beam expander 601c may be a cylindrical convex lens or a cylindrical concave lens. In an embodiment, a convex lens (not shown) may be used as a beam expander. It will be apparent to one of skill in the art that other lenses, lens systems or other optical systems or combinations thereof, can be used to spread and more evenly distribute the light.

Referring back to Figure 4, in an embodiment, the light bar guides 322 are angled inward toward the ultrasonic imaging plane on the end retaining the distal ends of the fibers. The inward angling of the distal end of the light bar guide 322 permits the light emitting there-from to better fill, and thus, evenly illuminate the optical window 203. Gap 402, which may include a beam expander, may provide space for the light transmitted across the light path 132 to expand to fill the optical window 203. The inward angling tends to cause the direction of the light incident on the surface of the volume 160 to strike the surface at an angle less than normal, and thus, potentially, to better propagate into the volume beneath the acoustic lens 205 covering the ultrasound transducers 310.

Turning back to Figure 1, because the probe 102 is intended for handheld use, the weight and flexibility of the light path 132, the electrical path 108 and the optional power and control lines 109 is of consideration. In an embodiment, to make the light path 132 lighter and more flexible, the light path 132 is constructed from as few fibers as possible. A limiting factor to how few a number of fibers that can be used, is the amount of light carried across the optical path 132. The transmission of too much light over a fiber will damage the fiber. The light path 132 must carry the total amount of light that will be fluent on the surface of the volume 160, plus any light lost (e.g., absorbed or scattered) between the light source 129 and the surface of the volume 160 illuminated. Since the maximum area of illumination is known not to exceed the size of the optical window 203, and because the area of illumination is subject to fluence limits per unit area, a total light energy carried by the light path 132 can be approximated by multiplying the fluence limit by the size of the optical windows 203. The FDA provides numbers for the human safe level of fluence.

The volume 160 illuminated generally has its own optoacoustic response, which is especially apparent where light fluence is greatest, namely, at the surface of the volume 160. Increasing the area of illumination onto the surface of the volume 160 (e.g., by increasing the size of the optical window 203 and beam) reduces the optoacoustic affect generated by the surface of the volume 160 itself, and thus may reduce the undesirable optoacoustic signal generated by the surface of the volume 160 itself as compared to a desired signal representing the inhomogenities 161, 162.

In addition to unwanted optoacoustic signal generated by the surface of the volume 160 itself, there may be other sources of unwanted optoacoustic signals that can be detected by the ultrasound transducer, such as the side walls surrounding the space between the optical windows 205 and the respective light bar guides 322, the acoustic lens 205 and portions of the transducer housing 316. The optical windows 203 and any optional beam expander 601b, 601c may also be sources of unwanted optoacoustic signals that can be detected by the ultrasound transducer.

In an embodiment, the walls surrounding the space between the optical windows 205 and the respective light bar guides 322 may be made from a material that has high acoustic absorption properties and/or that is white and/or has high light scattering and/or reflecting properties. Using materials having these characteristics may reduce unwanted optoacoustic signals that can be detected by the ultrasound transducer. In an embodiment, the spacers 322 can be made from a resin material such as Micro-Mark CR-600, a two part high performance casting resin that dries to a white color.

In an embodiment, a layer (not shown) of material that has high acoustic absorption properties and/or that is white and/or has high light scattering properties is placed between the transducer assembly 315 and the light bar guides 322 in the assembled probe 102. Alternatively, the layer may be applied directly to the transducer assembly 315 or the light bar guide 322 where the two parts contact in the assembled probe 102. This layer may reduce unwanted optoacoustic signals that can be detected by the ultrasound transducer. In an embodiment, the layer can be made from a resin material such as Micro-Mark CR-600, a two part high performance casting resin that dries to a white color. In an embodiment, the layer (not shown) may also comprise a reflective coating. In an embodiment a reflective coating of gold is applied to the layer to reflect light that might otherwise strike the layer.

In an embodiment, anti-reflective coatings may be used to reduce the optoacoustic signature of the optical window 203 and/or the beam expander 601b, 601c. In an embodiment, magnesium fluoride may be used as an anti-reflective coating on the optical window 203 and/or the beam expander 601b, 601c. Anti-reflective coatings may be used to reduce and/or minimize energy absorbed or reflected by the optical window 203.

In an embodiment, the optoacoustic signature of the transducer assembly 315 and/or acoustic lens 205 can be reduced by whitening. In an embodiment, an acoustic lens 205 comprising RTV silicon rubber may be whitened and have its optoacoustic signature reduced by being doped with about 4% TiO₂. It is believed that the TiO₂ doping increases the reflectivity of the acoustic lens and therefore the absorption, and also has a scattering effect that tends to diffuse the optoacoustic response of the RTV silicon rubber, bringing the response down to a lower frequency which can be more easily filtered. As discussed above, the outer surface of the transducer assembly 315 and/or acoustic lens 205 may be given a metal coating, such as gold, copper, aluminum or brass. In an embodiment, the metal coating, and in particular, gold, reduces the optoacoustic signature of the transducer assembly 315 and/or acoustic lens 205. It is believed that gold reduces the optoacoustic signature of the acoustic lens 205 because of its high reflectivity in the light spectrum.

As discussed above, the optical fibers at the end of the optical path 132 are retained by the light bar guide 322 with all of the fiber ends retained by the light bar guide 322 located on substantially the same plane. In an embodiment, the fiber ends may be fixed in place using mechanical force, an adhesive, or a combination of mechanical force and an adhesive. The fibers may be glued near their distal end to keep them in the desired location and pattern, and/or to reduce output of mechanical energy due to laser firing. In an embodiment, the spaces between optical fibers fixed within the light bar guide 322 may be filled with a material having one or more of the following characteristics: sound absorbing, light scattering, white and/or light reflecting. In an embodiment, the optical fibers, which may be encased by a light bar guide 322 at the distal end of the light path 132 are fused. Fusing fibers at the distal end of the light path 132 may permit the light emitting from the light path to be more uniform.

In an embodiment, a reflective coating is placed on areas of the shells 302, 304 where laser light emanating from the optical path 132 may strike it, including with the assembled probe, and in the areas designed to make skin contact, e.g., near the optical window 203 and other portions of the distal end of the probe 102. In an embodiment, the shells 302, 304 are coated in gold where laser light emanating from the optical path 132 may, or is likely to strike it. In an embodiment, portions of the shell 302, 304 may be made from gold, although at present this may be cost prohibitive.

In an embodiment, a proximity detector system (not shown) is used to determine that the distal end of the probe 102 is on or very near the surface of a volume. Among the reasons such a proximity detector system is desirable is that it can be used to prevent pulsing of the light source 129 when the probe 102 is not in close proximity to a volume 160 under inspection, or to be inspected. This may be a safety issue as the light source 129 may produce light at levels that can be harmful, e.g., to the eyes. The proximity detector system may be implemented in the form of: a mechanical contact switch at the distal end of the probe; an optical switch looking at reflections of a non-harmful beam from the surface of the volume 160; a conductive switch that is closed by contact with the volume 160 and/or any acoustic gel or other materials between the volume 160 and the distal end of the probe; a conductive switch and a standoff comprising a conductive surface for contact with the distal end of the probe 102; a conductive switch and a thin, optically and acoustically transparent, conductive surface applied to the surface of the volume 160 of interest; an acoustic transducer switch that can detect close proximity of the volume 160 by transmitting and looking for the reflection of a sound within a specific time; an acoustic transducer switch that can detect close proximity of the volume 160 by using a narrow shape sound transmitter and receiver and using the reflection to detect proximity; using one or more of the transducers in the transducer array as a proximity detector by looking for a signal return; or by operating the device 100 in an ultrasound mode and looking for an ultrasound image.

In an embodiment, an optical detector (not shown) may be located in the probe 102 to take a measurement from which output energy can be estimated or deduced. In an embodiment, the optical detector will measure reflected energy such as energy reflected by the beam expander or optical window. In an embodiment, the optical detector will measure scattered energy such as energy scattered by the materials surrounding the gap 402. The measurement of the optical detector can be transmitted to the system chassis 101 via control signal line 109, where it can be analyzed to deduce or estimate the light output of the probe 102. In an embodiment, control functionality in the system chassis 101 can control or regulate the light output of the light system 129, and thus the light output of the probe 102 based on a measurement made by the optical detector. In an embodiment, control functionality in the system chassis 101 can control or regulate the gain in the transducer receivers to compensate for variation of the light output of the probe 102 based on a measurement made by the optical detector. In an embodiment, the computing subsystem 128 can trigger differing activity from light system 129 over control signal line 106 based on a measurement made by the optical detector. In an embodiment, a measurement made by the optical detector can be used to control for variations in the electrical system or the power to the device 101. Similarly, in an embodiment, a measurement made by the optical detector can be used to control for variations in the optical path 132 or other optical elements of the device 100. In an embodiment, the optical detector can be used to cause the fluence of light output by the probe 102 to remain close to, but below, safe limits by accommodating for variations in electrical or optical characteristics that might otherwise cause the fluence of light output by the probe 102 to exceed or fall far below the safe limit.

The present system and methods are described above with reference to block diagrams and operational illustrations of methods and devices comprising an optoacoustic probe. It is understood that each block of the block diagrams or operational illustrations, and combinations of blocks in the block diagrams or operational illustrations, may be implemented by means of analog or digital hardware and computer program instructions. These computer program instructions may be provided to a processor of a general purpose computer, special purpose computer, ASIC, or other programmable data processing apparatus, such that the instructions, which execute via the processor of the computer or other programmable data processing apparatus, implements the functions/acts specified in the block diagrams or operational block or blocks. In some alternate implementations, the functions/acts noted in the blocks may occur out of the order noted in the operational illustrations. For example, two blocks shown in succession may in fact be executed substantially concurrently or the blocks may sometimes be executed in the reverse order, depending upon the functionality/acts involved.

While the present disclosure has been particularly shown and described with reference to a preferred embodiment thereof, it will be understood by those skilled in the art that various changes in form and details may be made therein without departing from the scope of the invention as defined in the appended claims.

## Claims

1. A handheld optoacoustic probe (102) having a distal end, the probe comprising:
a plurality of optical fibers comprising a first light path (132), the plurality of optical fibers having a first end formed into at least one fiber bundle providing an input, and having a second end formed from the distal end of at least some of the plurality of optical fibers;
a light bar guide (332) retaining the distal end of the at least some of the plurality of optical fibers on the same plane;
an optical window (203) associated with, and spaced from the light bar guide; a silicon rubber acoustic lens (205) having an inner surface and an outer surface at the distal end of the probe, the acoustic lens being whitened by doping;
an ultrasound transducer array (310) having an active end, the array comprising a plurality of ultrasound transducer elements, the ultrasound transducer array having the inner surface of the acoustic lens at its active end;
a reflective metal surrounding the outer surface of the acoustic lens; and a handheld probe shell (302, 304), the shell housing the light bar guide and the ultrasound transducer array, and housing the acoustic lens adjacent to the optical window at the distal end of the probe.

2. The handheld optoacoustic probe claimed in claim 1 wherein the transducer array is formed on a flex circuit (312), and a plurality of traces connect the transducer array to cable connectors, the optoacoustic probe further comprising an optically reflective and RF shielding material surrounding at least a substantial portion of the flex circuit and the cable connectors,
for example wherein the material is copper, and the copper is tied to an electrical ground,
for example further comprising a beam expander located between the distal end of the at least some of the plurality of optical fibers and the optical window, for example in which the beam expander comprises a ground glass element,
for example, wherein the beam expander comprises at least one optical lens.

3. The handheld optoacoustic probe of claim 1, wherein the silicon rubber acoustic lens comprises a room temperature vulcanization silicon rubber acoustic lens.

4. A handheld optoacoustic probe according to claim 1 wherein:
the distal end of the at least some of the plurality of optical fibers are lapped and polished to provide for a more uniform angle of light emission.

5. A handheld optoacoustic probe according to claim 1 wherein the optical window has an inner side and an outer side, and the optical window is arranged so that light emitted from the distal ends of the at least some of the plurality of optical fibers fills the outer side of the optical window with substantially uniform fluence;
wherein the handheld probe shell houses the light bar guide and the ultrasound transducer array, and housing optical window and the active end of the ultrasound transducer array near the distal end of the probe, where the optical window is housed in a manner that prevents contact between a coupling agent and the distal ends of the at least some of the plurality of optical fibers, thus mitigating a potential acoustic effect of the coupling agent in response to light emitting from the at least some of the plurality of optical fibers.

6. The handheld optoacoustic probe claimed in claim 5, further comprising at least one of:
a beam expander (601b, 601c) located between the distal ends of the at least some of the plurality of optical fibers and the optical window; and
a gap (402) between the distal ends of the at least some of the plurality of optical fibers and the optical window; and
for example wherein the optical window is configured to act as a beam expander to further distribute light emitted from the distal ends of the at least some of the plurality of optical fibers across the outer surface of the optical window with substantially uniform fluence, and
for example wherein the silicon rubber acoustic lens comprises a room-temperature-vulcanization silicon rubber acoustic lens.

7. A handheld optoacoustic probe according to claim 1 wherein:
the second end of the plurality of optical fibers is formed into first and second groups of optical fiber, each of the first and second groups of optical fiber consisting of approximately the same number of optical fibers; for example wherein the distal end of the first and second groups of fiber are fused;
the light bar guide is a first light bar guide associated with the first group of optical fibers and a second light bar guide is associated with the second group of optical fibers and respectively, each of the first and second light bar guides retaining a distal end of the respective group of optical fibers on the same plane; and the probe comprises
a first and second optical window associated with, and spaced from, each of the first and second light bar guides, respectively; and
an RTV silicon rubber acoustic lens having an inner surface and an outer surface, the acoustic lens being doped with TiO₂;
for example wherein the plurality of optical fibers comprising at least one of the at least one fiber bundles are fused.

8. The handheld optoacoustic probe of claim 7, wherein the reflective metal is selected from the group of: aluminum, copper, gold, silver, brass or noble metal, for example wherein the reflective metal is gold and the gold reflective metal surrounding the outer surface of the acoustic lens is one of:
(a) overlaid with a protective layer to avoid flaking or cracking of the reflective metal; and
(b) enveloped in a protective layer to avoid flaking or cracking, wherein the protective layer is transparent and has a low acoustic absorption.

9. The handheld optoacoustic probe of claim 8, further comprising:
an edge protector to protect at least one edge of the gold metal surrounding the outer surface of the acoustic lens to reduce flaking or cracking of the reflective metal at the at least one edge.

10. The handheld optoacoustic probe claimed in claim 1, further comprising:
a first beam expander located between the distal end of the first group of optical fibers and the first optical window, for example wherein the first beam expander is arranged to expand light beams emitted by the first group of optical fibers to fill the first optical window, for example wherein the space between the first optical window and the first light bar guide is defined on its sides by walls, and wherein the walls are made from one of: a material that has high acoustic absorption; and a material that has a high light scattering property.

11. The handheld optoacoustic probe claimed in claim 10, wherein the walls are made from one of: a white or light colored material, and a material having low optical absorption and high optical reflection.

12. The handheld optoacoustic probe claimed in claim 10, further comprising: a second beam expander located between the distal end of the second group of optical fibers and the second optical window.

13. The handheld optoacoustic probe of any preceding claim, wherein the first end is formed into a first fiber bundle for providing an input, the first fiber bundle providing a fixed position for each of the fibers therein with respect to the other fibers therein, and wherein the second end is formed into a second fiber bundle for providing an output, and the second fiber bundle provides a fixed position for each of the fibers therein with respect to the other fibers therein;
the position of a fiber in the first fiber bundle being randomly related to the position of the same fiber in the second end.

## Patentansprüche

1. Optoakustische Handsonde (102) mit einem distalen Ende, wobei die Sonde Folgendes umfasst:
mehrere optische Fasern mit einem ersten Lichtweg (132), wobei die mehreren optischen Fasern ein erstes Ende, das zu mindestens einem Faserbündel geformt ist, das einen Eingang bereitstellt, und ein zweites Ende aufweisen, das aus dem distalen Ende von mindestens einigen der mehreren optischen Fasern gebildet ist;
einen Lichtbalkenleiter (332), der das distale Ende der mindestens einigen der mehreren optischen Fasern in der gleichen Ebene hält;
ein optisches Fenster (203), das mit dem Lichtbalkenleiter assoziiert und davon beabstandet ist;
eine akustische Linse (205) aus Silikonkautschuk mit einer Innenfläche und einer Außenfläche am distalen Ende der Sonde, wobei die akustische Linse durch Dotieren geweißt ist;
ein Ultraschallwandler-Array (310) mit einem aktiven Ende, wobei das Array mehrere Ultraschallwandlerelemente umfasst, wobei das Ultraschallwandler-Array die Innenfläche der akustischen Linse an seinem aktiven Ende aufweist;
ein reflektierendes Metall, das die Außenfläche der akustischen Linse umgibt; und
ein Handsondengehäuse (302, 304), wobei das Gehäuse den Lichtbalkenleiter und das Ultraschallwandler-Array aufnimmt und die akustische Linse neben dem optischen Fenster am distalen Ende der Sonde aufnimmt.

2. Optoakustische Handsonde nach Anspruch 1, wobei das Wandlerarray auf einer flexiblen Schaltung (312) ausgebildet ist und mehrere Leiterbahnen das Wandlerarray mit Kabelverbindern verbinden, wobei die optoakustische Sonde ferner ein optisch reflektierendes und RF-Abschirmungsmaterial umfasst, das zumindest einen wesentlichen Teil der flexiblen Schaltung und der Kabelverbinder umgibt,
wobei zum Beispiel das Material aus Kupfer besteht und das Kupfer an eine elektrische Masse gebunden ist,
die zum Beispiel ferner einen Strahlaufweiter umfasst, der zwischen dem distalen Ende der mindestens einigen der mehreren optischen Fasern und dem optischen Fenster angeordnet ist, wobei zum Beispiel der Strahlaufweiter ein geschliffenes Glaselement umfasst,
wobei zum Beispiel der Strahlaufweiter mindestens eine optische Linse umfasst.

3. Optoakustische Handsonde nach Anspruch 1, wobei die akustische Linse aus Silikonkautschuk eine akustische Linse aus bei Raumtemperatur vulkanisiertem Silikonkautschuk umfasst.

4. Optoakustische Handsonde nach Anspruch 1, wobei:
das distale Ende der mindestens einigen der mehreren optischen Fasern geläppt und poliert ist, um einen gleichmäßigeren Lichtemissionswinkel zu schaffen.

5. Optoakustische Handsonde nach Anspruch 1, wobei das optische Fenster eine Innenseite und eine Außenseite aufweist und das optische Fenster so angeordnet ist, dass Licht, das von den distalen Enden der mindestens einigen der mehreren optischen Fasern emittiert wird, die Außenseite des optischen Fensters mit im Wesentlichen gleichmäßiger Fluenz ausfüllt;
wobei das Handsondengehäuse den Lichtbalkenleiter und das Ultraschallwandler-Array aufnimmt und das optische Fenster und das aktive Ende des Ultraschallwandler-Arrays in der Nähe des distalen Endes der Sonde aufnimmt, wobei das optische Fenster in einer Weise aufgenommen wird, die Kontakt zwischen einem Kopplungsmittel und den distalen Enden der zumindest einigen der mehreren optischen Fasern verhindert, wodurch ein potenzieller akustischer Effekt des Kopplungsmittels als Reaktion auf Licht, das von den zumindest einigen der mehreren optischen Fasern emittiert wird, abgeschwächt wird.

6. Optoakustische Handsonde nach Anspruch 5, die ferner mindestens eines umfasst aus
einem Strahlaufweiter (601b, 601c), der zwischen den distalen Enden der mindestens einigen der mehreren optischen Fasern und dem optischen Fenster angeordnet ist; und
einem Spalt (402) zwischen den distalen Enden der zumindest einigen der mehreren optischen Fasern und dem optischen Fenster; und
wobei zum Beispiel das optische Fenster so konfiguriert ist, dass es als Strahlaufweiter wirkt, um Licht, das von den distalen Enden der mindestens einigen der mehreren optischen Fasern emittiert wird, mit im Wesentlichen gleichmäßiger Fluenz über die Außenfläche des optischen Fensters weiter zu verteilen, und
wobei zum Beispiel die akustische Linse aus Silikonkautschuk eine akustische Linse aus bei Raumtemperatur vulkanisiertem Silikonkautschuk umfasst.

7. Optoakustische Handsonde nach Anspruch 1, wobei:
das zweite Ende der mehreren optischen Fasern in eine erste und eine zweite Gruppe von optischen Fasern geformt ist, wobei die erste und zweite Gruppe von optischen Fasern jeweils aus etwa der gleichen Anzahl von optischen Fasern besteht; wobei zum Beispiel das distale Ende der ersten und zweiten Gruppe von Fasern verschmolzen ist;
der Lichtbalkenleiter ein erster Lichtbalkenleiter ist, der mit der ersten Gruppe optischer Fasern assoziiert ist, und ein zweiter Lichtbalkenleiter ist mit der zweiten Gruppe optischer Fasern assoziiert, wobei jeder der ersten und zweiten Lichtbalkenleiter ein distales Ende der jeweiligen Gruppe von optischen Fasern in der gleichen Ebene hält; und die Sonde Folgendes umfasst:
ein erstes und zweites optisches Fenster, die mit dem ersten bzw. dem zweiten Lichtbalkenleiter assoziiert und davon beabstandet sind, und
eine akustische Linse aus RTV-Silikonkautschuk mit einer Innenfläche und einer Außenfläche, wobei die akustische Linse mit TiO₂ dotiert ist;
wobei zum Beispiel die mehreren optischen Fasern, die mindestens eines der mindestens einen Faserbündel umfassen, verschmolzen sind.

8. Optoakustische Handsonde nach Anspruch 7, wobei das reflektierende Metall ausgewählt ist aus der Gruppe bestehend aus Aluminium, Kupfer, Gold, Silber, Messing und Edelmetall, wobei zum Beispiel das reflektierende Metall Gold ist und das die Außenfläche der akustischen Linse umgebende goldene reflektierende Metall eines ist aus:
(a) mit einer Schutzschicht überzogen, um Abblättern oder Reißen des reflektierenden Metalls zu vermeiden; und
(b) von einer Schutzschicht umhüllt, um Abblättern oder Reißen zu vermeiden, wobei die Schutzschicht transparent ist und eine geringe akustische Absorption hat.

9. Optoakustische Handsonde nach Anspruch 8, die ferner Folgendes umfasst:
einen Kantenschutz, um mindestens eine Kante des die Außenfläche der akustischen Linse umgebenden Goldmetalls zu schützen, um Abblättern oder Reißen des reflektierenden Metalls an der mindestens einen Kante zu verringern.

10. Optoakustische Handsonde nach Anspruch 1, die ferner Folgendes umfasst:
einen ersten Strahlaufweiter, der zwischen dem distalen Ende der ersten Gruppe von optischen Fasern und dem ersten optischen Fenster angeordnet ist, wobei zum Beispiel der erste Strahlaufweiter so angeordnet ist, dass er Lichtstrahlen aufweitet, die von der ersten Gruppe von optischen Fasern emittiert werden, um das erste optische Fenster zu füllen, wobei zum Beispiel der Raum zwischen dem ersten optischen Fenster und dem ersten Lichtbalkenleiter an seinen Seiten durch Wände definiert ist, und wobei die Wände aus einem Material mit einer hohen akustischen Absorption oder einem Material mit einer hohen Lichtstreueigenschaft hergestellt sind.

11. Optoakustische Handsonde nach Anspruch 10, wobei die Wände aus einem weißen oder hell gefärbten Material oder einem Material mit geringer optischer Absorption und hoher optischer Reflexion hergestellt sind.

12. Optoakustische Handsonde nach Anspruch 10, die ferner einen zweiten Strahlaufweiter umfasst, der zwischen dem distalen Ende der zweiten Gruppe von optischen Fasern und dem zweiten optischen Fenster angeordnet ist.

13. Optoakustische Handsonde nach einem der vorherigen Ansprüche, wobei das erste Ende zu einem ersten Faserbündel ausgebildet ist, um einen Eingang bereitzustellen, wobei das erste Faserbündel eine feste Position für jede der Fasern darin in Bezug auf die anderen Fasern darin bereitstellt, und wobei das zweite Ende zu einem zweiten Faserbündel ausgebildet ist, um einen Ausgang bereitzustellen, und das zweite Faserbündel eine feste Position für jede der Fasern darin in Bezug auf die anderen Fasern darin bereitstellt;
die Position einer Faser in dem ersten Faserbündel in zufälliger Beziehung zu der Position der gleichen Faser in dem zweiten Ende steht.

## Revendications

1. Sonde opto-acoustique portative (102) ayant une extrémité distale, la sonde comprenant :
une pluralité de fibres optiques comprenant un premier trajet de lumière (132), la pluralité de fibres optiques ayant une première extrémité formée en au moins un faisceau de fibres fournissant une entrée, et ayant une deuxième extrémité formée à partir de l'extrémité distale d'au moins quelques-unes de la pluralité de fibres optiques ;
un guide de barre de lumière (332) retenant l'extrémité distale des au moins quelques-unes de la pluralité de fibres optiques sur le même plan ;
une fenêtre optique (203) associée au guide de barre de lumière, et espacée de celui-ci ;
une lentille acoustique en caoutchouc de silicium (205) ayant une surface interne et une surface externe au niveau de l'extrémité distale de la sonde, la lentille acoustique étant blanchie par dopage ;
un réseau de transducteurs ultrasonores (310) ayant une extrémité active, le réseau comprenant une pluralité d'éléments transducteurs ultrasonores, le réseau de transducteurs ultrasonores ayant la surface interne de la lentille acoustique au niveau de son extrémité active ;
un métal réfléchissant entourant la surface externe de la lentille acoustique ; et
une enveloppe de sonde portative (302, 304), l'enveloppe recevant le guide de barre de lumière et le réseau de transducteurs ultrasonores, et recevant la lentille acoustique adjacente à la fenêtre optique au niveau de l'extrémité distale de la sonde.

2. Sonde opto-acoustique portative selon la revendication 1 dans laquelle le réseau de transducteurs est formé sur un circuit souple (312), et une pluralité de traces connectent le réseau de transducteurs à des connecteurs de câble, la sonde opto-acoustique comprenant en outre un matériau de blindage RF et réfléchissant optiquement qui entoure au moins une partie substantielle du circuit souple et des connecteurs de câble,
par exemple dans laquelle le matériau est le cuivre, et le cuivre est relié à une masse électrique,
par exemple comprenant en outre un dilatateur de faisceau situé entre l'extrémité distale des au moins quelques-unes de la pluralité de fibres optiques et la fenêtre optique, par exemple dans laquelle le dilatateur de faisceau comprend un élément en verre dépoli, par exemple, dans laquelle le dilatateur de faisceau comprend au moins une lentille optique.

3. Sonde opto-acoustique portative selon la revendication 1, dans laquelle la lentille acoustique en caoutchouc de silicium comprend une lentille acoustique en caoutchouc de silicium à vulcanisation à température ambiante.

4. Sonde opto-acoustique portative selon la revendication 1 dans laquelle :
les extrémités distales des au moins quelques-unes de la pluralité de fibres optiques sont superposées et polies pour fournir un angle plus uniforme d'émission de lumière.

5. Sonde opto-acoustique portative selon la revendication 1 dans laquelle la fenêtre optique a un côté interne et un côté externe, et la fenêtre optique est agencée de sorte que la lumière émise à partir des extrémités distales des au moins quelques-unes de la pluralité de fibres optiques remplit le côté externe de la fenêtre optique avec une fluence substantiellement uniforme ;
dans laquelle l'enveloppe de sonde portative reçoit le guide de barre de lumière et le réseau de transducteurs ultrasonores, et reçoit la fenêtre optique et l'extrémité active du réseau de transducteurs ultrasonores près de l'extrémité distale de la sonde, où la fenêtre optique est reçue d'une manière qui empêche tout contact entre un agent de couplage et les extrémités distales des au moins quelques-unes de la pluralité de fibres optiques, atténuant ainsi un effet acoustique potentiel de l'agent de couplage en réponse à l'émission de lumière à partir des au moins quelques-unes de la pluralité de fibres optiques.

6. Sonde opto-acoustique portative selon la revendication 5, comprenant en outre au moins l'un parmi :
un dilatateur de faisceau (601b, 601c) situé entre les extrémités distales des au moins quelques-unes de la pluralité de fibres optiques et la fenêtre optique ; et
un écartement (402) entre les extrémités distales des au moins quelques-unes de la pluralité de fibres optiques et la fenêtre optique ; et
par exemple dans laquelle la fenêtre optique est configurée pour agir comme un dilatateur de faisceau pour distribuer davantage la lumière émise à partir des extrémités distales des au moins quelques-unes de la pluralité de fibres optiques sur toute la surface externe de la fenêtre optique avec une fluence substantiellement uniforme, et
par exemple dans laquelle la lentille acoustique en caoutchouc de silicium comprend une lentille acoustique en caoutchouc de silicium à vulcanisation à température ambiante.

7. Sonde opto-acoustique portative selon la revendication 1 dans laquelle :
la deuxième extrémité de la pluralité de fibres optiques est formée en des premier et deuxième groupes de fibre optique, chacun des premier et deuxième groupes de fibre optique constitué d'approximativement le même nombre de fibres optiques ; par exemple dans laquelle les extrémités distales des premier et deuxième groupes de fibres sont fusionnées ;
le guide de barre de lumière est un premier guide de barre de lumière associé au premier groupe de fibres optiques et un deuxième guide de barre de lumière est associé au deuxième groupe de fibres optiques et respectivement, chacun des premier et deuxième guides de barre de lumière retenant une extrémité distale du groupe respectif de fibres optiques sur le même plan ; et la sonde comprend
des première et deuxième fenêtres optiques associées à chacun des premier et deuxième guides de barre de lumière et espacées de ceux-ci, respectivement ; et
une lentille acoustique en caoutchouc de silicium à vulcanisation à température ambiance ayant une surface interne et une surface externe, la lentille acoustique étant dopée avec du TiO₂ ;
par exemple dans laquelle la pluralité de fibres optiques composant au moins l'un des au moins un faisceau de fibres sont fusionnées.

8. Sonde opto-acoustique portative selon la revendication 7, dans laquelle le métal réfléchissant est choisi parmi le groupe comprenant : l'aluminium, le cuivre, l'or, l'argent, le laiton ou un métal noble, par exemple dans laquelle le métal réfléchissant est l'or et le métal or réfléchissant entourant la surface externe de la lentille acoustique est l'un parmi :
(a) recouvert d'une couche protectrice pour éviter l'écaillage ou la fissuration du métal réfléchissant ; et
(b) enveloppé d'une couche protectrice pour éviter l'écaillage ou la fissuration, dans laquelle la couche protectrice est transparente et a une faible absorption acoustique.

9. Sonde opto-acoustique portative selon la revendication 8, comprenant en outre :
un protecteur de bord pour protéger au moins un bord du métal or entourant la surface externe de la lentille acoustique pour réduire l'écaillage ou la fissuration du métal réfléchissant au niveau de l'au moins un bord.

10. Sonde opto-acoustique portative selon la revendication 1, comprenant en outre :
un premier dilatateur de faisceau situé entre l'extrémité distale du premier groupe de fibres optiques et la première fenêtre optique, par exemple dans laquelle le premier dilatateur de faisceau est agencé pour dilater des faisceaux de lumière émis par le premier groupe de fibres optiques pour remplir la première fenêtre optique, par exemple dans laquelle l'espace entre la première fenêtre optique et le premier guide de barre de lumière est défini sur ses côtés par des parois, et dans laquelle les parois sont faites à partir de l'un : d'un matériau qui a une absorption acoustique élevée ; et d'un matériau qui a une propriété de diffusion de lumière élevée.

11. Sonde opto-acoustique portative selon la revendication 10, dans laquelle les parois sont fabriquées à partir de l'un : d'un matériau blanc ou de couleur claire, et d'un matériau ayant une faible absorption optique et une réflexion optique élevée.

12. Sonde opto-acoustique portative selon la revendication 10, comprenant en outre :
un deuxième dilatateur de faisceau situé entre l'extrémité distale du deuxième groupe de fibres optiques et la deuxième fenêtre optique.

13. Sonde opto-acoustique portative selon l'une quelconque des revendications précédentes, dans laquelle la première extrémité est formée en un premier faisceau de fibres destiné à fournir une entrée, le premier faisceau de fibres fournissant une position fixe pour chacune des fibres dans celui-ci par rapport aux autres fibres dans celui-ci, et dans laquelle la deuxième extrémité est formée en un deuxième faisceau de fibres destiné à fournir une sortie, et le deuxième faisceau de fibres fournit une position fixe pour chacune des fibres dans celui-ci par rapport aux autres fibres dans celui-ci ;
la position d'une fibre dans le premier faisceau de fibres étant liée de manière aléatoire à la position de la même fibre dans la deuxième extrémité.
